# EUROPÄISCHE PATENTSCHRIFT

(11) **EP 3 534 770 B1**
(45) Veröffentlichungstag und Bekanntmachung des Hinweises auf die Patenterteilung: **24.07.2024**
(21) Anmeldenummer: 17792029.5
(22) Anmeldetag: 27.10.2017
(51) Int. Cl.: A61B 1/018, A61B 1/00

(54) **ARBEITSKANALELEMENT, ENDOSKOP MIT EINEM ARBEITSKANALELEMENT, UND VERFAHREN ZUR ANWENDUNG EINES ARBEITSKANALS IN EINEM ENDOSKOP**
WORKING CHANNEL ELEMENT, ENDOSCOPE HAVING A WORKING CHANNEL ELEMENT, AND METHOD FOR USING A WORKING CHANNEL IN AN ENDOSCOPE
ÉLÉMENT À CANAL DE TRAVAIL, ENDOSCOPE AYANT UN ÉLÉMENT À CANAL DE TRAVAIL ET PROCÉDÉ POUR UILISER UN CANAL DE TRAVAIL DANS UN ENDOSCOPE

(30) Priorität: 04.11.2016 DE 102016121056
(43) Veröffentlichungstag der Anmeldung: 11.09.2019
(73) Patentinhaber: Digital Endoscopy GmbH, 86316 Friedberg (DE)
(72) Erfinder: DO, Anh Minh, 86316 Friedberg (DE); SCHRÖTER, Tilman, 86316 Friedberg (DE)
(74) Vertreter: TBK
(86) Internationale Anmeldenummer: PCT/EP2017/077588
(87) Internationale Veröffentlichungsnummer: WO 2018/083024

(56) Entgegenhaltungen:
- JP-B1- 6 017 741
- JP-B2- 3 595 409
- US-A1- 2006 149 129
- US-A1- 2006 252 993
- US-A1- 2011 213 300
- US-A1- 2012 265 132
- US-A1- 2015 057 537
- US-A1- 2016 227 988

## Beschreibung

Die vorliegende Erfindung bezieht sich auf ein Arbeitskanalelement für ein Endoskop, und Endoskop mit diesem Arbeitskanalelement. Ferner bezieht sich die vorliegende Erfindung auf ein Verfahren zur Anwendung eines Arbeitskanals in einem Endoskop.

Ein solches Endoskop kann beispielsweise ein Duodenoskop sein, also ein Endoskop zur Untersuchung z.B. der Speiseröhre oder auch des Duodenums, des Gallengangs, der Galle, des Bauchspeicheldrüsengangs, der Bauchspeicheldrüse etc. Mit Hilfe des Duodenoskops ist es möglich, durch die Speiseröhre, den Magen und den Magenausgang in das Duodenum zu gelangen.

Das Duodenoskop hat eine zur Seite gerichtete (laterale) Optik (Beleuchtungseinrichtung und Kamera). Dies kann das Einführen und Vorschieben durch die Speiseröhre zu erschweren, da keine "nach vorn" erfolgende Betrachtung ohne weiteres möglich ist. Lediglich im Magen oder im Duodenum besteht ausreichend Raum, um das distale Ende des Duodenoskops um etwa 90° abzuwinkeln, um so nach vorn schauen zu können.

Das Duodenoskop hat außerdem am Ausgang des Arbeitskanals einen Albarranhebel, der durch Verschwenken eine gezielte Umlenkung der Werkzeuge ermöglicht, die durch den Arbeitskanal geschoben werden. Ein solcher Albarranhebel hat in der Regel eine komplexe Geometrie, die schwer zu reinigen ist.

Bei einigen Duodenoskopen kann der Albarranhebel als Einmalverwendungselement gestaltet werden, um die Möglichkeit des Anhaftens von Keimen am wiederverwendbaren Endoskop noch besser zu vermeiden.

Selbst bei diesen Duodenoskopen können Keime sich im Arbeitskanal verfangen.

Nach der Anwendung des Duodenoskops wird dieses einer Aufbereitung unterzogen. Diese muss zuverlässig die Übertragung sämtlicher Mikroorganismen wie Bakterien, Viren, Pilze, Würmer aber auch Sporen ausschließen. Bei der Aufbereitung wird zunächst das Duodenoskop inklusive Arbeitskanal manuell gereinigt, um organisches Material oder chemische Rückstände rückstandsfrei zu entfernen. Nach der Reinigung erfolgt eine maschinelle Desinfektion oder Sterilisation.

Die US 2015/057537 A1 offenbart ein Endoskop, in das ein Schlauchelement als ein Arbeitskanalelement eingeführt wird. Dieses Arbeitskanalelement hat die Merkmale des Oberbegriffs von Anspruch 1.

Die US 2006/149129 A1 zeigt ein Endoskop, in das ein Betrachtungsrohr eingeführt wird.

Die US 2012/0265132 A1 zeigt einen Katheter, der ein Multi-lumen-Rohr aufweist. In das Multi-lumen-Rohr können Elemente eingeführt werden können.

Die US 2016/0227988 A1 offenbart ein Endoskop, in das beispielweise eine Biopsiezange eingeführt wird.

Die JP 6 017741 B1 offenbart ein Endoskop mit einem Einführschlauch. Durch das Endoskop werden Behandlungselemente in einen Patienten eingeführt.

Die JP 3 595489 B2 zeigt ein Endoskop, in das ein Arbeitskanalelement eingeführt wird. Das Arbeitskanalelement hat einen proximalen Zugang.

US 2011/213300 A1 offenbart ein kleineres Endoskop, das in ein größeres Endoskop eingeführt wird. Der Griffabschnitt des kleineren Endoskops ist am Griffabschnitt des größeren Endoskops befestigt.

US 2006/252993 A1 offenbart eine Befestigung einer Hilfsvorrichtung an einem Endoskop, wobei eine Montagevorrichtung um den Außenumfang eines Zugangs am Endoskop angeordnet ist.

Es ist die Aufgabe der vorliegenden Erfindung, ein verbessertes Arbeitskanalelement und ein verbessertes Endoskop zu schaffen, bei dem das Weitergeben von Keimen noch besser vermieden wird.

Diese Aufgabe ist durch ein Arbeitskanalelement mit den Merkmalen von Anspruch 1 gelöst. Ein Endoskop mit dem Arbeitskanalelement ist in Anspruch 4 aufgezeigt. Ein Verfahren zur Anwendung eines Arbeitskanals in einem Endoskop ist in Anspruch 5 aufgezeigt.

Vorteilhafte Weiterbildungen sind Gegenstand der abhängigen Ansprüche.

Ein erfindungsgemäßes Arbeitskanalelement laut Anspruch 1 weist eine Rohrform mit einem Innenumfang und einem Außenumfang auf, wobei durch die Rohrform ein Instrument schiebbar ist. Das Arbeitskanalelement ist daran angepasst, mit seiner distalen Seite voran in einen Arbeitskanal eines Endoskops vorübergehend eingeführt zu werden. Das Arbeitskanalelement weist eine Außenwand auf, die lateral von einem Instrumentenzugang durchdrungen ist. Durch den Instrumentenzugang können Instrumente in einen Innenkanal des Arbeitskanalelements geschoben werden.

Das Arbeitskanalelement hat ein Außenmaß, das ein Einführen in einen Arbeitskanal eines Endoskops erlaubt. Das Arbeitskanalelement ist an die Form des Arbeitskanals des Endoskops angepasst. Die Form des Arbeitskanalelements ist vorzugsweise eine runde Form. Das Außenmaß (z.B. Außendurchmesser) des Arbeitskanalelements ist somit geringfügig kleiner als das Innenmaß (z.B. Innendurchmesser) des Arbeitskanals des Endoskops.

Somit kann in der Erfindung ein Endoskop mit einem nicht fest verbundenen, entfernbaren Arbeitskanal versehen werden.

Dadurch ergibt sich eine vereinfachte Reinigung des Endoskops, da für jeden Patienten ein eigener Arbeitskanal verwendet werden kann, der entsorgt werden kann. Gereinigt braucht nur das Endoskop werden, in dessen eigenen Arbeitskanal lediglich das erfindungsgemäße Arbeitskanalelement eingeführt worden war.

Das Arbeitskanalelement weist an der distalen Seite einen Abwinkelungsabschnitt auf. Somit kann das Arbeitskanalelement selbst die Aufgabe des Auslenkens eines eingeschobenen Instrumentes übernehmen, die z.B. ein bisheriger Albarranhebel inne hatte. Die bisherige komplexe Geometrie eines Albarranhebels am distalen Ende des Endoskops kann dabei entfallen. Somit wird auch das Endoskop selbst leichter und besser reinigbar. Kreuzkontaminationen von Patient zu Patient können noch besser verhindert werden.

Das Arbeitskanalelement weist an der proximalen Seite eine Steuereinheit zum Steuern des Abwinkelns des Abwinkelungsabschnittes auf. Die Steuereinheit ist so aufgebaut, dass sie im Arbeitskanalelement zwischen Innenumfang und Außenumfang geführte am Abwinkelungsabschnitt verankerte Steuerdrähte spannt, um das Abwinkeln des Abwinkelungsabschnittes zu steuern. Dadurch kann das Auslenken eines eingeschobenen Instrumentes vorteilhaft und einfach bewerkstelligt werden.

Die Steuereinheit ist als Schieber aufgebaut ist, in dem das proximale Ende der Steuerdrähte verankert ist.

Die Steuereinheit kann eine Vielzahl an separaten Steuerelementen aufweisen, wobei jedem separaten Steuerelement mindestens einer der Steuerdrähte zugeordnet ist.

Das Arbeitskanalelement weist der proximalen Seite eine Feststelleinrichtung zum Fixieren einer abgewinkelten Stellung des distalen Abwinkelungsabschnittes auf. Durch die Feststelleinrichtung (z.B. ein Bremsmechanismus) kann der Arzt dann die für ihn ideale ausgelenkte Position fixieren und Therapieinstrumente durch den Arbeitskanal an die gewünschte Position im Gewebe bringen.

Das Arbeitskanalelement kann an der proximalen Seite eine Montageeinrichtung aufweisen, durch die das Arbeitskanalelement an einem Endoskop montiert werden kann. Dadurch kann das erfindungsgemäße Arbeitskanalelement schnell und stabil am Endoskop auf eine Weise montiert werden, die dem Bediener (Arzt) vertraut ist. Die Montageeinrichtung des erfindungsgemäßen Arbeitskanalelements ist vorzugsweise distal einer Steuereinheit des Arbeitskanalelements vorgesehen.

Ein Endoskop kann mit einem solchen Arbeitskanalelement ausgestattet sein. Die Steuereinheit zum Steuern des Abwinkelns des Abwinkelungsabschnittes kann nahe zu einem Griffabschnitt des Endoskops so anordenbar sein, dass der Bedienende bequem sowohl den Steuerabschnitt des Endoskops als auch die Steuereinheit zum Steuern des Abwinkelns des Abwinkelungsabschnittes (idealerweise mit einer Hand) erreichen und bedienen kann. Das Endoskop kann ein beliebiges Endoskop sein, welches einen eigenen Arbeitskanal besitzt.

In einem erfindungsgemäßen Verfahren zur Anwendung eines Arbeitskanals in einem Endoskop wird ein separates Arbeitskanalelement mit seiner distalen Seite voran in einen Arbeitskanal eines Endoskops vorübergehend eingeführt.

Das Verfahren kann des Weiteren die folgenden Schritte aufweisen: Einführen des Arbeitskanalelements in das Endoskop; und Abwinkeln eines distalen Endes des Arbeitskanalelementes zur gewünschten Position.

Nach der einmaligen Anwendung des Arbeitskanalelementes kann das Arbeitskanalelement aus dem Endoskop herausgezogen und entsorgt werden.

Nach der einmaligen Anwendung des Arbeitskanalelementes kann das Arbeitskanalelement aus dem Endoskop an der distalen Seite des Endoskops herausgezogen und entsorgt werden. Das benutzte Arbeitskanalelement verlässt das Endoskop an der distalen Seite des Endoskops. Somit werden Keime nicht in die proximale Richtung im Endoskop geleitet. Dadurch kann ein noch besserer Schutz vor Kontamination geschaffen werden.

Die vorstehend erläuterten Aspekte der vorliegenden Erfindung können geeignet kombiniert werden.

### Kurzbeschreibung der Zeichnungen

Fig. 1 zeigt eine perspektivische Ansicht eines erfindungsgemäßen Arbeitskanalelementes eines ersten Ausführungsbeispiels in einem im Endoskop eingebauten Zustand.
Fig. 2 zeigt eine perspektivische Ansicht des erfindungsgemäßen Arbeitskanalelementes des ersten Ausführungsbeispiels separat vom Endoskop, wobei die Lage eines Ultraschallsensors angedeutet ist.
Fig. 3 zeigt eine perspektivische Ansicht einer Steuereinheit des erfindungsgemäßen Arbeitskanalelementes des ersten Ausführungsbeispiels.
Fig. 4 zeigt eine perspektivische Ansicht eines proximalen Endabschnittes des erfindungsgemäßen Arbeitskanalelementes des ersten Ausführungsbeispiels.
Fig. 5 zeigt eine weitere perspektivische Ansicht des proximalen Endabschnittes des erfindungsgemäßen Arbeitskanalelementes des ersten Ausführungsbeispiels.
Fig. 6 zeigt eine perspektivische Ansicht eines Steuerelements der Steuereinheit des erfindungsgemäßen Arbeitskanalelementes des ersten Ausführungsbeispiels.
Fig. 7 zeigt eine perspektivische Ansicht von Elementen einer Montageeinrichtung des erfindungsgemäßen Arbeitskanalelementes des ersten Ausführungsbeispiels.
Fig. 8 zeigt eine perspektivische Ansicht eines nicht erfindungsgemäßen Arbeitskanalelementes eines fünften Ausführungsbeispiels.
Fig. 9 zeigt eine perspektivische Ansicht des ersten Anwendungsbeispiels des nicht erfindungsgemäßen Arbeitskanalelementes des fünften Ausführungsbeispiels im am Endoskop angebauten Zustand.
Fig. 10 zeigt eine perspektivische Ansicht eines nicht erfindungsgemäßen Arbeitskanalelementes eines sechsten Ausführungsbeispiels.
Fig. 11 zeigt eine perspektivische Ansicht des zweiten Anwendungsbeispiels des nicht erfindungsgemäßen Arbeitskanalelementes des sechsten Ausführungsbeispiels im am Endoskop angebauten Zustand.
Fig. 12 zeigt eine perspektivische Ansicht des distalen Endabschnittes des erfindungsgemäßen Arbeitskanalelementes des ersten Ausführungsbeispiels. Nachstehend ist die vorliegende Erfindung detailliert unter Bezugnahme auf die Zeichnungen anhand von Ausführungsbeispielen beschrieben.

### Ausführungsbeispiel 1

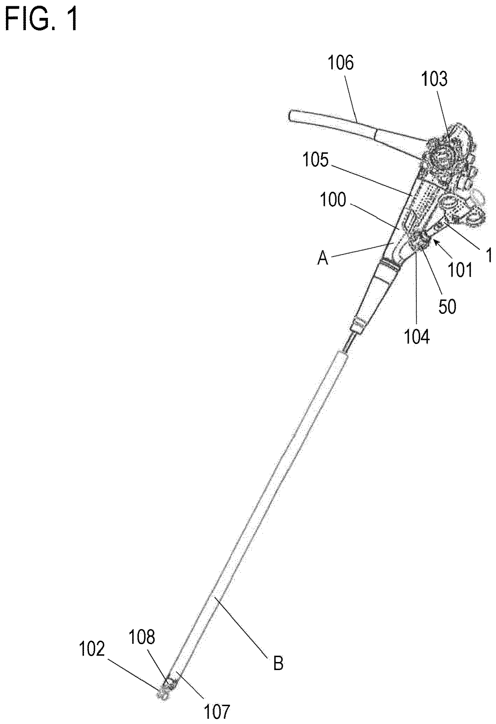

Zunächst ist unter Bezugnahme auf die Figuren 1 bis 7 ein erstes Ausführungsbeispiel der vorliegenden Erfindung beschrieben.

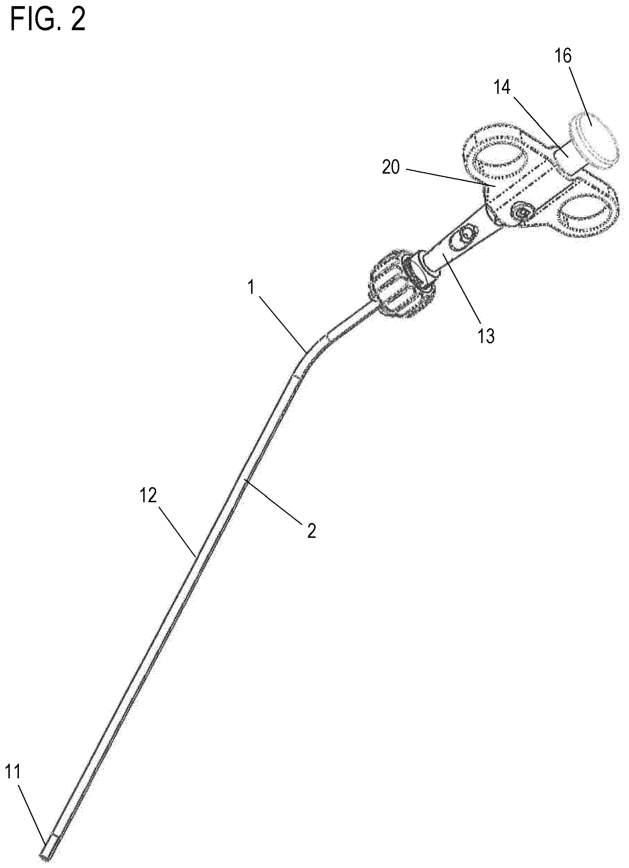

Die Figuren 1 und 2 zeigen jeweils eine perspektivische Ansicht eines ersten Ausführungsbeispiels eines erfindungsgemäßen Arbeitskanalelementes 1. Genauer gesagt zeigt Fig. 1 einen Zustand, bei dem das erfindungsgemäße Arbeitskanalelement 1 in einem Endoskop eingebaut ist, und Fig. 2 zeigt das Arbeitskanalelement 1 separat vom Endoskop.

Zunächst ist unter Bezugnahme auf Figur 1 ein Endoskop 100 kurz beschrieben, bei dem das erfindungsgemäße Arbeitskanalelement 1 angewendet werden kann. Das Endoskop 100 hat einen länglichen Endoskopkörper.

Dieses Endoskop 100 ist z. B. als flexibles Endoskop für den Magen-Darm-Trakt ausgebildet. Das Endoskop weist ein Bedienteil A und einen Einführabschnitt B auf. Der Bedienteil A befindet sich an der proximalen Seite und der Einführabschnitt B befindet sich an der distalen Seite des Endoskops 100. Der Bedienteil A weist einen Arbeitskanaleinlass 101 und einen Stellknopf 103 für das Biegen eines Endoskop-Abwinklungsabschnittes am distalen Ende des Einführabschnitts B des Endoskops auf. Der Arbeitskanaleinlass 101 ist mit einem Anschlusselement 104 versehen, das in der Form eines üblichen Luer-lock-Anschlusses ausgebildet sein kann. Ferner ist der Bedienteil A mit einem Griffabschnitt 105 versehen, an dem der Bedienende das Endoskop 100 hält.

Der Bedienteil A ist per Kabel 106 ist mit einem Videoprozessor, einer Lichtquellenvorrichtung und einer Anzeigevorrichtung und dergleichen verbunden.

Der Einführabschnitt B ist ein langes schlauchartiges Element. Das proximale Ende des Einführabschnitts ist mit dem Bedienteil A verbunden. Der Einführabschnitt B weist einen flexiblen Abschnitt und den Endoskop-Abwinklungsabschnitt 107 in dieser Reihenfolge vom Bedienteil A aus gesehen auf. Der flexible Abschnitt ist elastisch. Der Endoskop-Abwinklungsabschnitt 107 wird im Ansprechen auf eine Betätigung des Stellknopfes 103 abgewinkelt. Am distalen Ende des Endoskop-Abwinklungsabschnittes 107 ist ein starrer Endstückabschnitt ausgebildet. Der starre Endstückabschnitt bildet den sogenannten Endoskopkopf 108. Ein Ultraschallsensor 102 ist am distalen Ende des Endoskopkopfes 108 angeordnet.

An der distalen Seite des Griffabschnittes 105 besitzt der Bedienteil A einen abgewinkelten Zugang.

Im Endoskop 1 ist ein Arbeitskanal vorgesehen. Der Arbeitskanal beginnt am Arbeitskanaleinlass 101, der an der proximalen Seite des abgewinkelten Zugangs ausgebildet ist, durchläuft den abgewinkelten Zugang, mündet in den Bedienteil A und durchläuft den Bedienteil A in distaler Richtung, durchläuft den Einführabschnitt B und mündet am Endoskopkopf 108 so, dass er in die distale Richtung gerichtet ist.

In diesen Arbeitskanal des Endoskops 1 ist das nachstehend beschriebene erfindungsgemäße Arbeitskanalelement 1 einführbar.

Das Arbeitskanalelement 1 ist in Figur 2 separat vom Endoskop gezeigt.

Das Arbeitskanalelement 1 weist ein flexibles Schlauchelement 2 auf. Das flexible Schlauchelement 2 ist aus einem elastischem Material hergestellt und besteht vorzugsweise aus Kunststoff oder Gummi. Das flexible Schlauchelement 2 ist als ein zylinderartiges Rohr mit einem Innenumfang und einem Außenumfang ausgebildet. Der Außenumfang des flexiblen Schlauchelements 2 ist kleiner als der vorstehend beschriebene Arbeitskanal des Endoskops 1. Der Innenumfang des flexiblen Schlauchelements 2 hat eine derartige Größe, dass Mikrowerkzeuge zur Untersuchung z.B. der Speiseröhre oder auch des Duodenums, des Gallengangs, der Galle, des Bauchspeicheldrüsengangs, der Bauchspeicheldrüse etc. hindurchgeführt werden können.

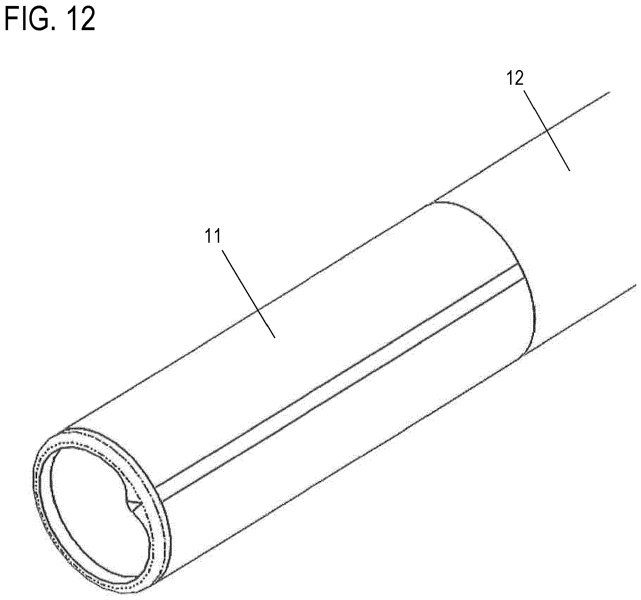

Das flexible Schlauchelement 2 ist an der proximalen Seite als Hauptschlauch 12 ausgebildet. An der distalen Seite des Schlauchelements 2 hat das Arbeitskanalelement 1 einen Abwinkelungsabschnitt 11, der sich distal an den Hauptschlauch 12 anschließt, siehe auch Fig. 12.

Der Abwinkelungsabschnitt 11 ist ein biegbarer Abschnitt des Schlauchelements 2. An seinem proximalen Ende besitzt der Abwinkelungsabschnitt 11 ein proximales Ringelement, an dem das distale Ende des Hauptschlauches 12 angeschlossen ist. An dem distalen Ende besitzt der Abwinkelungsabschnitt 11 ein distales Ringelement. Zwischen dem proximales Ringelement und dem distalen Ringelement ist der Abwinkelungsabschnitt 11 über nachstehend beschriebene Zugdrähte (Seilzüge) biegbar (abwinkelbar).

In den Zeichnungen nicht gezeigte Zugdrähte (Seilzüge) sind zwischen dem Innenumfang und dem Außenumfang des Schlauchelements 2 im Arbeitskanalelement 1 sich in der Längsrichtung erstreckend angeordnet. Im Ausführungsbeispiel wird zumindest ein Zugdraht angewendet. Um die Funktion des Abwinkelungsabschnittes 11 zu gewährleisten, ist ein Zugdraht ausreichend. Um eine feinere (genauere) Steuerung der Abwinklung des Abwinkelungsabschnittes 11 zu erzielen, können mehrere Zugdrähte angewendet werden.

Die Zugdrähte des Arbeitskanalelements 1 verlaufen in Zugdrahtkanälen oder Zugdrahtbereichen zwischen dem Innenumfang und dem Außenumfang des Schlauchelements 2. Diese Zugdrahtkanäle oder Zugdrahtbereiche sind zumindest an der distalen Seite des Arbeitskanalelements 1 gegenüber der Umgebung abgedichtet.

Im vorliegenden Ausführungsbeispiel ist ein Zugdraht im erfindungsgemäßen Arbeitskanalelement 1 vorgesehen. An der distalen Seite des Schlauchelements 2, also im Abwinkelungsabschnitt 11, ist der Zugdraht durch das proximalen Ringelement und das Schlauchstück des Abwinkelungsabschnittes 11 zum distalen Ringelement geführt und am distalen Ringelement verankert. Anders ausgedrückt bildet das distale Ringelement den distalen Verankerungspunkt des Zugdrahtes. Der Zugdraht ist somit entlang der axialen Richtung des Arbeitskanalelements 1 angeordnet und an der distalen Seite des Abwinkelungsabschnittes 11 verankert und kann an der proximalen Seite des Arbeitskanalelements 1 durch eine Steuereinheit betätigt werden. D.h. bei Ziehen des Zugdrahtes wird der Verankerungspunkt an der distalen Seite des Abwinkelungsabschnittes 11 des Arbeitskanalelements 1 in proximale Richtung gezogen, wodurch der Abwinkelungsabschnitt 11 gebogen wird.

Somit kann durch Ziehen des Zugdrahtes in bekannter Weise die laterale Ausrichtung des Abwinkelungsabschnittes 11 geändert werden. Anders ausgedrückt ändert bei im Endoskop 100 eingeführtem Arbeitskanalelement 1 der Abwinkelungsabschnitt 11 den Ausrichtungswinkel der Mikrowerkzeuge, die durch den Innenkanal 13 des Schlauchelements 2 geschoben werden. Die Ausrichtung der Mikrowerkzeuge kann somit durch Ändern des Winkels des Abwinkelungsabschnittes 11 relativ zum Endoskopkopf 108 zu der erwünschten lateralen Richtung geändert werden. Die Mikrowerkzeuge stehen dann seitlich vom Endoskopkopf 108 in der gewählten Winkelstellung vor, um in z.B. einen Gallengang geschoben zu werden.

Im ersten Ausführungsbeispiel befindet sich bei im Endoskop 100 eingeführtem Arbeitskanalelement 1 das distale Ende des Arbeitskanalelements 1 proximal vom Ultraschallsensor 102.

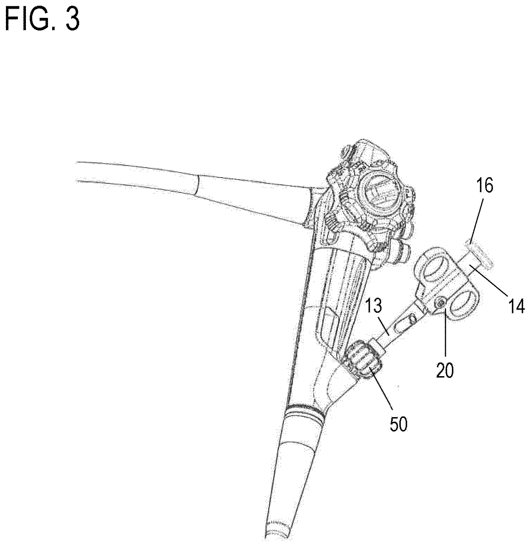

Nachstehend ist unter Bezugnahme auf die Figuren 3 bis 6 erläutert, durch welchen Aufbau der Abwinkelungsabschnitt 11 abgewinkelt und somit gesteuert werden kann.

Die proximale Seite des Arbeitskanalelements 1 ist in Figur 3 gezeigt.

An der proximalen Seite des Schlauchelements 2 hat das Arbeitskanalelement 1 ein Übergangselement 13. Das Übergangselement 13 ist als Hohlelement mit in proximaler Richtung sich vergrößerndem Durchmesser ausgebildet.

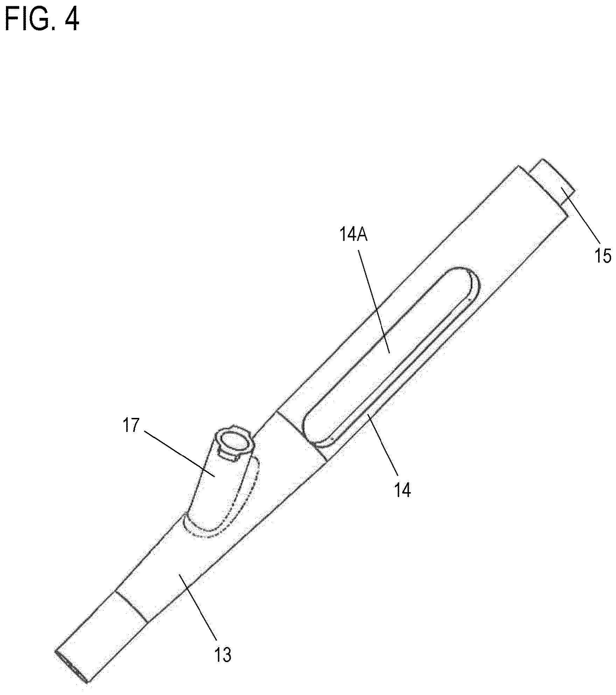

Proximal vom Übergangselement 13 ist ein Stabelement 14 vorgesehen. Das Stabelement 14 ist ein mit einem Boden versehener Hohlzylinder, der einen sich in Längsrichtung des Stabelements 14 sich erstreckenden durchgehenden lateralen Schlitz 14A aufweist, wie dies in Fig. 4 gezeigt ist. Der Boden des Stabelements 14 weist in proximale Richtung. Die dem Boden gegenüberliegende Öffnung des Stabelements 14 ist mit dem Übergangselement 13 verbunden.

Das proximale Ende des Schlauchelements 2, das Übergangselement 13 und das Stabelement 14 sind auf gleicher Achse angeordnet.

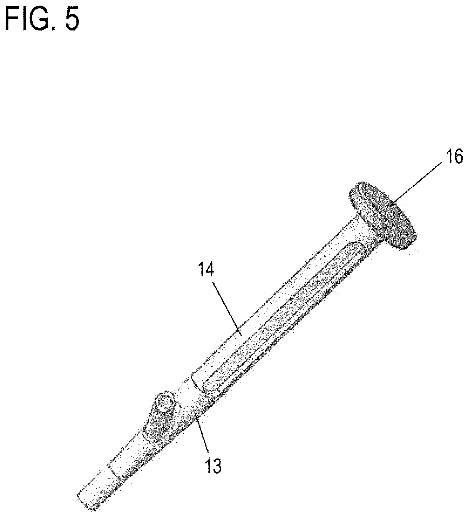

Am Boden des Stabelements 14 ist eine Nase 15 angeordnet, die in proximaler Richtung weist. Auf der Nase 15 ist ein Tellerelement 16 aufgesetzt, dessen proximale Fläche als Daumenbetätigungsfläche dient, siehe Figur 5.

Die jeweilige Verbindung zwischen Schlauchelement 2 und Übergangselement 13, Übergangselement 13 und Stabelement 14, Tellerelement 16 und Nase 15 kann durch Kleben, durch Laserstrahlbehandlung, durch Löten oder durch ein anderes geeignetes Verfahren erzeugt werden. Das Tellerelement 16 kann auf die Nase 15 auch aufgesteckt oder aufgepresst werden.

An der Außenwand des Übergangselements 13 erstreckt sich lateral vom Übergangselement 13 ein die Außenwand des Übergangselements 13 durchdringender rohrartiger Instrumentenzugang 17, durch den Instrumente, Mikrowerkzeuge etc. in den Innenkanal des Schlauchelements 2, also des Arbeitskanalelements 1, in distale Richtung geschoben werden können. Der Instrumentenzugang 17 kann an dem Übergangselement 13 durch Laserstrahlbehandlung, durch Löten oder durch ein anderes geeignetes Verfahren angebracht werden.

Im proximalen Endbereich des Schlauchelements 2 oder im Bereich des Übergangselements 13 durchdringt der Zugdraht (alternativ die Zugdrähte) den Innenumfang des Arbeitskanalelements 1 und läuft zur nachstehend beschriebenen Steuereinheit 20. Um eine Beeinträchtigung mit einzuführenden Instrumenten zu vermeiden, kann der Ort, an dem der Zugdraht den Innenumfang des Arbeitskanalelements 1 durchdringt, sich proximal vom Instrumentenzugang 17 befinden.

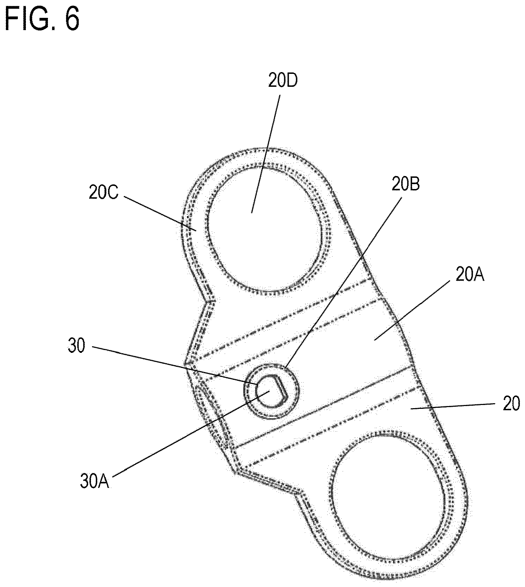

Auf dem Stabelement 14 ist ein Schieber 20 als Steuereinheit in Längsrichtung des Stabelements 14 verschiebbar angeordnet. Nachstehend ist der Schieber 20 anhand Figur 6 genauer erläutert.

Der Schieber 20 hat einen Rohrabschnitt 20A mit einem Innendurchmesser, der geringfügig größer als der Außendurchmesser des Stabelements 14 ist. Dadurch kann der Schieber 20 auf dem Stabelement 14 verschoben werden.

Der Schieber 20 hat im Bereich des Rohrabschnitts 20A ein Loch 20B, das sich senkrecht zur Längsachse des Rohrabschnitts 20A erstreckt und in die radiale, also laterale, Richtung weist.

Ferner ragen radial vom Rohrabschnitt 20A zwei Seitenflügel 20C flanschartig vor. Die beiden Seitenflügel 20C erstrecken sich in zueinander entgegengesetzte Richtungen vom Rohrabschnitt 20A. das vorstehend erwähnte Loch 20B ist unter einem Winkel von 90 Grad zu dem jeweiligen Seitenflügel 20C versetzt angeordnet, wie dies in Fig. 6 gezeigt ist.

Jeder Seitenflügel 20C hat eine Öffnung 20D in einer Größe, die ein Hindurchschieben eines Fingers des Bedieners erlaubt.

Am Schieber 20 ist der Zugdraht verankert. Somit bildet der Schieber 20 den proximalen Verankerungspunkt des Zugdrahtes (der Zugdrähte). Der Zugdraht ist z.B. am Innenumfang des Schiebers 20 verankert.

Im Loch 20B des Rohrabschnitts 20A des Schiebers 20 ist ein Drehbolzen 30 als Feststelleinrichtung eingesetzt. Der Drehbolzen 30 ist als einseitig abgeflachter Zylinderstift ausgebildet, der im Querschnitt die Form eines Buchstaben D bildet. An der nicht abgeflachten Seite hat der Drehbolzen 30 einen Außendurchmesser, der größer als die Spaltbreite des Schlitzes 14A ist. Im Querschnitt des Drehbolzens 30 beträgt Maß von der abgeflachten Seite zu dem gegenüberliegenden Außenumfang weniger als die Spaltbreite des Schlitzes 14A. Anders ausgedrückt hat der Drehbolzen 30 ein erstes Radialmaß, das größer als die Spaltbreite des Schlitzes 14A ist, und ein zweites Radialmaß, das kleiner als die Spaltbreite des Schlitzes 14A ist.

Der Drehbolzen 30 ragt in den Schlitz 14A hinein. Der Drehbolzen 30 hat an der nach außen weisenden Seite eine Vertiefung 30A, in die ein Werkzeug formschlüssig eingesetzt werden kann, um den Drehbolzen 30 zu drehen.

Bei Verschieben des Schiebers 20 auf dem und relativ zum Stabelement 14 hat der Drehbolzen 30 eine Relativstellung zum Schieber 20, bei der der Drehbolzen 30 mit seinem zweiten Radialmaß, das kleiner als die Spaltbreite des Schlitzes 14A ist, im Schlitz 14A geführt wird.

Somit kann durch Drehen des Drehbolzens 30 eine gewählte Längsverschiebeposition des Schiebers 20 relativ zum Stabelement 14 arretiert werden, indem der Drehbolzen 30 relativ zum Schieber 20 so weit gedreht wird, dass sein erstes Radialmaß an den Seitenwänden des Schlitzes 14A wirksam ist.

Das Arbeitskanalelement 1 kann, wenn es im Endoskop platziert ist, am Endoskop mittels einer Montageeinrichtung 50 montiert werden. Das Endoskop 100 selbst weist zu diesem Zweck an seinem Anschlusselement 104 einen üblichen Luer-lock-Anschluss auf, an dem die Montageeinrichtung 50 des Arbeitskanalelement 1 in Eingriff gelangt.

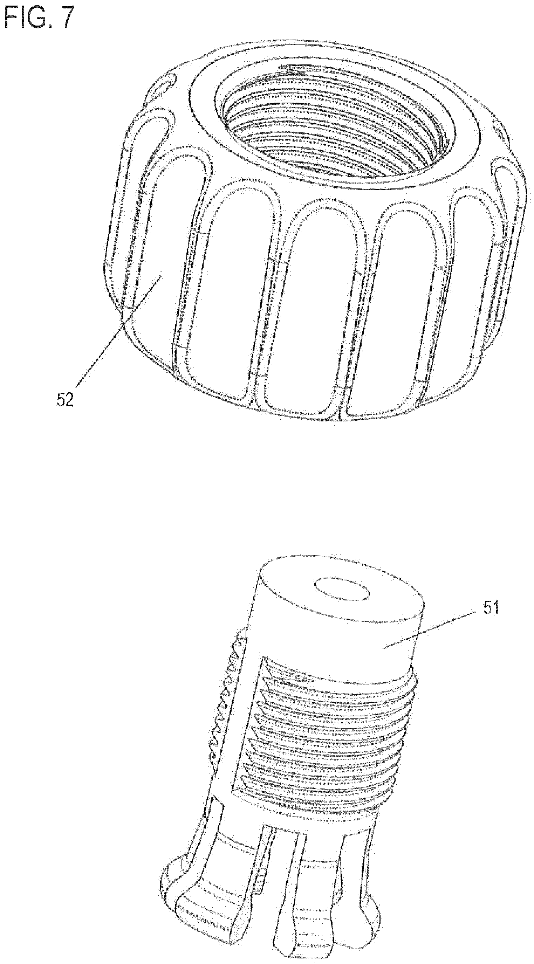

Fig. 7 zeigt die Elemente der Montageeinrichtung 50. Genauer gesagt besteht die Montageeinrichtung 50 aus einem zum Luer-lock-Anschluss des Endoskops 100 passenden Luer-lock-Gegenstück 51 und einer auf das Luer-lock-Gegenstück 51 aufgesetzten Schraubkappe 52.

Das Luer-lock-Gegenstück 51 sitzt am Außenumfang des Arbeitskanalelements 1 ungefähr in dem Bereich, in dem der Schlauch 2 (Hauptschlauch 12) und das Übergangselement 13 zusammentreffen. Das Luer-lock-Gegenstück 51 sitzt fest am Außenumfang des Arbeitskanalelements 1. Per Schraubkappe 52 wird das Luer-lock-Gegenstück 51 am Luer-lock-Anschluss des Endoskops 100 arretiert.

Da ein solches Luer-lock-System ein genormtes Verbindungssystem für Schlauchsysteme im medizinischen Bereich ist, muss es hier nicht genauer erläutert werden.

Bei einer Zugbewegung des Schiebers 20 in die proximale Richtung wird der Abwinkelungsabschnitt 11 an der lateralen Seite, an der der Zugdraht distal verankert ist, nach außen abgewinkelt.

Das Arbeitskanalelement 1 wird so in das Endoskop 100 eingeschoben, dass die radiale Seite des Abwinkelungsabschnittes 11, an der der distale Verankerungspunkt des Zugdrahtes angeordnet ist, sich an der zum Ultraschallkopf 102 entgegengesetzten radialen Seite befindet.

### Funktion der Erfindung

In der vorliegenden Erfindung wird somit ein vom Endoskop 100 separates Arbeitskanalelement 1 in den Arbeitskanal 101 des Endoskops 100 eingeführt. Genauer gesagt wird das Arbeitskanalelement 1 in den Arbeitskanal 101 von der proximalen Seite des Arbeitskanals 101 mit der distalen Seite des Arbeitskanalelements 1 voran eingeführt.

Nachdem das Arbeitskanalelement 1 in den Arbeitskanal 101 des Endoskops 100 vollständig eingeführt ist, wird das Arbeitskanalelement 1 durch die Montageeinrichtung 50 (Luer-lock) am Endoskop 100 montiert. In dieser Montagestellung weist die radiale Seite des Abwinkelungsabschnittes 11, in der der Zugdraht verankert ist, zu der zum Ultraschallkopf 102 entgegengesetzten radialen Seite.

Somit ist das mit dem Arbeitskanalelement 1 bestückte Endoskop 100 einsatzbereit. Das mit dem Arbeitskanalelement 1 bestückte Endoskop 100 wird in den Patienten zu dem erwünschten Anwendungsort (z.B. der Gallengang) eingeführt.

Nun kann der Bediener durch Verschieben des Schiebers 20 in die proximale Richtung den Abwinkelungsabschnitt 11 am distalen Ende des Arbeitskanalelementes 1 zu einer gewünschten Position abwinkeln. Wenn die gewünschte abgewinkelte Position des Abwinkelungsabschnittes 11 erreicht ist (z.B. gegenüberliegend dem Gallengang), wird die Stellung des Schiebers 20 durch die Feststelleinrichtung 30 arretiert.

Nun kann ein Instrument (Mikrowerkzeug) durch das Arbeitskanalelement 1 hindurchgeführt werden und die erforderliche Tätigkeit mit dem Instrument kann ausgeführt werden.

Nach der Beendigung der erforderlichen Tätigkeit wird das Instrument zurückgeführt. Nun wird der Schieber 20 gelöst. Durch Verschieben des Schiebers 20 in die distale Richtung wird der Zugdraht entspannt, womit sich der Abwinkelungsabschnitt 11 streckt. Das Endoskop kann vom Patienten entfernt werden.

Nun wird die Montageeinrichtung 50 gelöst und das Arbeitskanalelement 1 wird aus dem Endoskop 100 wieder herausgezogen und entsorgt.

Somit wird das Arbeitskanalelement 1 lediglich vorübergehend im Endoskop 100 angeordnet. Nach der einmaligen Anwendung des Arbeitskanalelementes 1 wird es aus dem Endoskop 100 wieder entfernt und entsorgt. Das Endoskop 100 wird hingegen der Wiederaufbereitung zugeführt.

### Wirkungen der Erfindung

Das erfindungsgemäße Arbeitskanalelement 1 kann bei Endoskopen sowohl die Funktion eines herkömmlichen Albarranhebels, der die Winkelstellung von Instrumenten einstellt, als auch eine horizontale Feinjustierung der gewünschten Zielposition des Instruments übernehmen. Hierbei ist das steuerbare Endstück mit einem Arbeitskanal verbunden, der selbst nicht fest mit dem Endoskop verbunden ist und aus dem Endoskop entfernbar ist.

Der große Vorteil an diesem System ist eine vereinfachte Reinigung des Endoskops und eine Verhinderung von Kreuzkontaminationen, da für jeden Patienten ein eigener Arbeitskanal verwendet wird und die komplexe Geometrie eines herkömmlichen Albarranhebels für die Reinigung komplett entfällt.

### Ausführungsbeispiel 2

Im vorliegenden zweiten Ausführungsbeispiel, das in den Zeichnungen nicht gezeigt ist, ist das Endoskop ein flexibles Endoskop mit Albarranhebel. In das Endoskop ist wie im ersten Ausführungsbeispiel ein erfindungsgemäßes Arbeitskanalelement 1 einführbar. Das Arbeitskanalelement 1 entspricht dem Arbeitskanalelement 1 des ersten Ausführungsbeispiels.

Das Endoskop besitzt zusätzlich zum im ersten Ausführungsbeispiel beschriebenen Aufbau einen (nicht gezeigten) Steuermechanismus (z.B. einen Joystick) zum Betätigen eines am distalen Ende des Einführabschnitts B angeordneten (nicht gezeigten) Albarranhebels.

Das erfindungsgemäße Arbeitskanalelement 1 zweiten Ausführungsbeispiels ragt, nachdem des in das Endoskop eingeführt und per Montageeinrichtung am Endoskop montiert worden ist, an der distalen Seite vom distalen Ende des Endoskopkopfes vor. Genauer gesagt ragt der Abwinkelungsabschnitt 11 des Arbeitskanalelements 1 vom distalen Ende des Endoskopkopfes vor. Der restliche Aufbau entspricht dem Aufbau des ersten Ausführungsbeispiels.

Im vorliegenden zweiten Ausführungsbeispiel kann die Abwinkelung der eingeführten Instrumente an der distalen Seite nicht nur durch lediglich den Abwinkelungsabschnitt 11 des Arbeitskanalelements 1 bewerkstelligt werden. Der Albarranhebel kann die Hauptabwinkelung des distalen Endstückes des Arbeitskanalelements 1 übernehmen. Zusätzlich zu dieser Hauptabwinkelung kann der Bedienende eine Feinjustierung durch eine separate Abwinkelung des Abwinkelungsabschnitt 11 des Arbeitskanalelements 1 vornehmen.

Dazu ist es ausreichend, dass zumindest ein Teil des Abwinkelungsabschnittes 11 bei im Endoskop eingebautem Arbeitskanalelement 1 distal vom Albarranhebel des Endoskops angeordnet ist.

Dadurch kann die Steuerbarkeit der eingeführten Instrumente stark verbessert werden.

### Ausführungsbeispiel 3

Im ersten Ausführungsbeispiel ist das Endoskop 1 ein flexibles Endoskop und das Arbeitskanalelement 1 weist den Abwinkelungsabschnitt 11 auf, der durch die Steuereinheit 20 abgewinkelt werden kann.

Im vorliegenden dritten Ausführungsbeispiel, das in den Zeichnungen nicht gezeigt ist, ist das Endoskop ebenfalls ein flexibles Endoskop. Das Endoskop weist ein Arbeitskanalelement 1 ohne Abwinkelungsabschnitt 11 und ohne Steuereinheit 20 auf.

Im Endoskop wird also ein Arbeitskanalelement 1 eingeführt, das selbst nicht gesteuert wird.

Auch bei diesem Aufbau ergibt sich die vorteilhafte Wirkung, dass nach einer Anwendung das erfindungsgemäße Arbeitskanalelement 1 aus dem Endoskop herausgezogen und entsorgt werden kann. Das Endoskop wird dem Reinigungsprozess übergeben.

Somit kann auch im dritten Ausführungsbeispiel vermieden werden, dass Keime aus früherer Anwendung am zu reinigenden Endoskop im Arbeitskanalbereich verbleiben.

### Ausführungsbeispiel 4

Im ersten Ausführungsbeispiel wird nach der Anwendung das Arbeitskanalelement 1 aus dem Endoskop 100 in proximaler Richtung wieder herausgezogen. Die Erfindung ist aber nicht darauf beschränkt.

Im vorliegenden vierten Ausführungsbeispiel wird das Arbeitskanalelement 1 im Bereich des proximalen Endes des Hauptschlauches 12 oder im Bereich des Übergangselements 13 durchtrennt (z.B. durchgeschnitten). Lediglich die Steuereinheit des Arbeitskanalelements 1 wird in proximaler Richtung entfernt. Der kontaminierte Teil des Arbeitskanalelements 1, also der Abwinkelungsabschnitt 11 und ein distaler Teil des Hauptschlauches 12, wird in distaler Richtung aus dem Endoskop 100 herausgezogen.

Somit kann ein noch besserer Schutz gegen Kreuzkontaminationen gewährleistet werden, da das Innere des Arbeitskanals 101 des Endoskops 100 mit Keimen nicht in Berührung gelangt.

In einer Untervariante des vierten Ausführungsbeispiels ist im Bereich des proximalen Endes des Hauptschlauches 12 oder im Bereich des Übergangselements 13 eine Sollbruchstelle vorgesehen. An der Sollbruchstelle kann das Arbeitskanalelement 1 abgeknickt und durchgebrochen werden. Dadurch wird das Trennen des Arbeitskanalelements 1 zwischen der in proximaler Richtung zu entfernenden Steuereinheit des Arbeitskanalelements 1 und dem in distaler Richtung aus dem Endoskop 100 zu entfernenden kontaminierten Teil des Arbeitskanalelements 1 vereinfacht.

Zudem kann die Steuereinheit des Arbeitskanalelements 1 in dieser Untervariante wiederverwendet werden.

### Ausführungsbeispiel 5

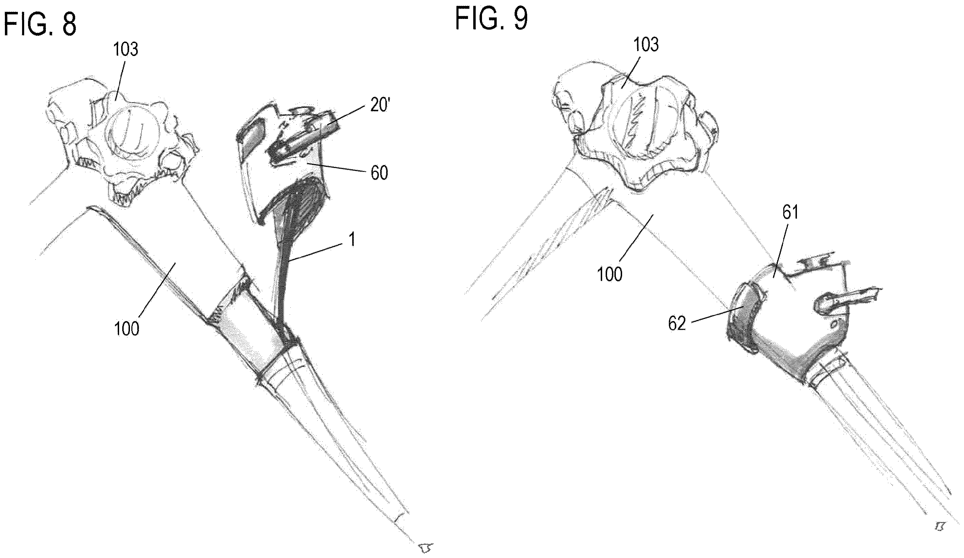

Ein weiteres Ausführungsbeispiel ist in den Figuren 8 und 9 gezeigt.

Figur 8 zeigt ein Arbeitskanalelement 1 des fünften Ausführungsbeispiels in einer Situation, in der es gerade an einem Endoskop 100 angebracht wird. Figur 9 zeigt das Arbeitskanalelement 1 des fünften Ausführungsbeispiels in einer Situation, in der die Montage des Arbeitskanalelements 1 an dem Endoskop 100 vollendet ist.

Das Arbeitskanalelement 1 des fünften Ausführungsbeispiels hat eine gegenüber der Steuereinheit 20 des ersten Ausführungsbeispiels abgewandelte Steuereinheit 20'. Anstelle des Schiebers 20 ist hier die Steuereinheit als Hebel 20' ausgebildet. Der Hebel 20' besitzt einen Fußbereich, an dem die Steuerdrähte so anmontiert sind, dass sie bei Ausführen einer Schwenkbewegung des Hebels 20' gespannt werden.

Der proximale Bereich des Arbeitskanalelements 1 des fünften Ausführungsbeispiels ist in einem Gehäuseteil 60 integriert, von dessen Außenfläche sich der Hebel 20' erstreckt.

Das Gehäuseteil 60 weist ein Gehäusestück 61 auf, das einen Bereich besitzt, der an die Außenkontur eines Griffabschnittes des Endoskops 100 so angepasst ist, dass er an den Griffabschnitt des Endoskops 100 aufgeklippt werden kann. Das Gehäusestück 61 hat an einer geeigneten Stelle ein Verschlusselement 62, durch das das Gehäusestück 61 am Endoskop 100 gesichert wird, siehe Fig. 9. Das Verschlusselement ist nicht auf ein Verschlusselement 62 wie in Fig. 9 beschränkt. Beliebige Arten und Formen eines Verschlusselementes sind anwendbar.

Im Inneren des Gehäusestücks 61 ist der proximale Bereich des Arbeitskanalelements 1 integriert. Beispielsweise kann der Hebel 20' eine (nicht gezeigte) Welle haben. Um diese Welle kann der Hebel 20' schwenken. Diese Welle ist durch einen Abschnitt des Arbeitskanalelements 1 gesteckt, der dem Stabelement 14 des ersten Ausführungsbeispiels entspricht. Im Inneren dieses Abschnittes des Arbeitskanalelements 1 sind um die Welle die Zugdrähte gewickelt. Somit können die Zugdrähte bei Ausführen einer Schwenkbewegung des Hebels 20' gespannt werden.

Beliebige andere Spannmöglichkeiten der Zugdrähte durch den Hebel 20' sind hier anwendbar.

Im fünften Ausführungsbeispiel kann das Arbeitskanalelement 1 nach dem Einstecken in den Arbeitskanal des Endoskops 100 leicht und schnell am Griffbereich des Endoskops 100 befestigt werden. Für den Bedienenden sind sowohl die Steuereinheit 103 des Endoskops 100 als auch der Hebel 20' leicht erreichbar und betätigbar.

Ein solches Endoskop wie es im fünften Ausführungsbeispiel genutzt wird, kann so gestaltet sein, dass sein distales Ende nur mittels des aufgeklippten Arbeitskanalelements 1 betätigbar ist. Somit kann ein solches Endoskop nicht ohne aufgeklipptes Arbeitskanalelement 1 benutzt werden.

### Ausführungsbeispiel 6

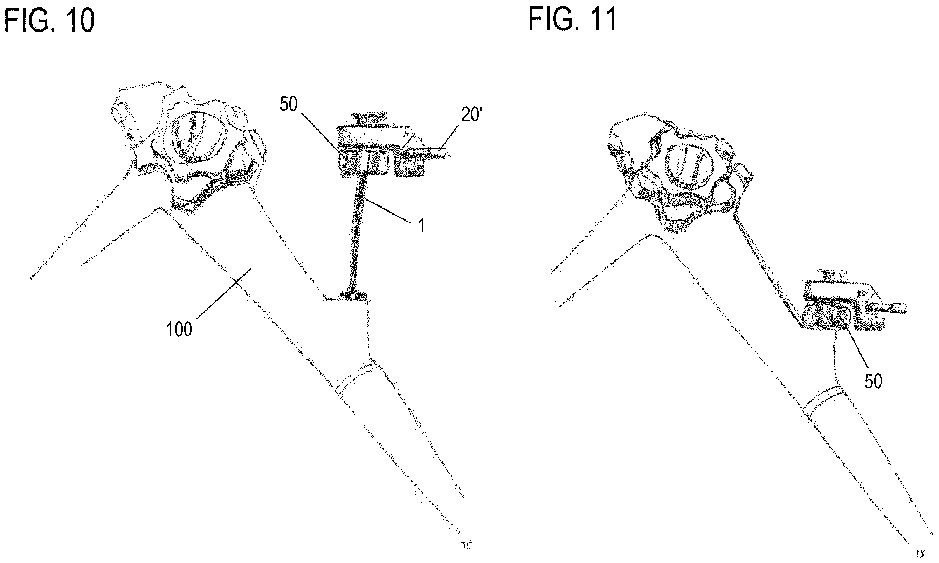

Ein weiteres Ausführungsbeispiel ist in den Figuren 10 und 11 gezeigt.

Figur 10 zeigt ein Arbeitskanalelement 1 des sechsten Ausführungsbeispiels in einer Situation, in der es gerade an einem Endoskop 100 angebracht wird. Figur 11 zeigt das Arbeitskanalelement 1 des sechsten Ausführungsbeispiels in einer Situation, in der die Montage des Arbeitskanalelements 1 an dem Endoskop 100 vollendet ist.

Das Arbeitskanalelement 1 des sechsten Ausführungsbeispiels hat eine gegenüber der Steuereinheit 20 des ersten Ausführungsbeispiels abgewandelte Steuereinheit, die ähnlich wie im fünften Ausführungsbeispiel als Hebel 20' ausgebildet ist.

Der proximale Bereich des Arbeitskanalelements 1 des sechsten Ausführungsbeispiels wird wie im ersten Ausführungsbeispiel per Luer-lock am Endoskop 100 montiert und gesichert.

Auch im sechsten Ausführungsbeispiel kann das Arbeitskanalelement 1 nach dem Einstecken in den Arbeitskanal des Endoskops 100 leicht und schnell per Luer-lock am Griffbereich des Endoskops 100 befestigt werden. Für den Bedienenden sind sowohl die Steuereinheit 103 des Endoskops 100 als auch der Hebel 20' leicht erreichbar und betätigbar.

Ein solches Endoskop wie es im sechsten Ausführungsbeispiel gezeigt ist, kann so gestaltet sein, dass sein distales Ende auch ohne Arbeitskanalelement 1 betätigbar ist. Somit kann ein solches Endoskop auch ohne anmontiertes Arbeitskanalelement 1 benutzt werden.

Die vorstehend erläuterten Ausführungsbeispiele können geeignet kombiniert werden.

### Weitere Ausführungsbeispiele und Alternativen

Die vorliegende Erfindung kann vorzugsweise bei einem Duodenoskop wie beschrieben angewendet werden, ist aber nicht darauf beschränkt. Die vorliegende Erfindung kann bei einem Gastroskop, einem Colonoskop oder einem ähnlichen Endoskop ebenfalls angewendet werden. Das Prinzip der Erfindung kann aber auch bei jeder anderen Art an Endoskop angewendet werden.

Im den Ausführungsbeispiel ist das Endoskop ein flexibles Endoskop. Das Endoskop kann auch als ein starres Endoskop ausgeführt sein, in welchem das erfindungsgemäße Arbeitskanalelement 1 anwendbar ist.

Im ersten Ausführungsbeispiel ist der Ultraschallsensor 102 am distalen Ende des Endoskopkopfes 108 angeordnet. Der Ultraschallsensor 102 kann auch weggelassen werden.

In einer Alternative können im ersten Ausführungsbeispiel zwei Zugseile im Arbeitskanalelement 1 vorgesehen werden, die relativ nahe beieinander an einer Seite des Umfangs am distalen Ende des Arbeitskanalelements 1 verankert sind. Die Zugdrähte können jeweils so angeordent sein, dass sie nicht um 180° Grad zueinander beabstandet sind, d.h. die Zugdrähte sind entlang des Umfangs des Schlauchelements 2 an einer Seite des Umfangs nahe zueinander angeordnet. An der distalen Seite des Schlauchelements 2, also im Abwinkelungsabschnitt 11, sind die Zugdrähte durch das proximalen Ringelement und das Schlauchstück des Abwinkelungsabschnittes 11 zum distalen Ringelement geführt und am distalen Ringelement verankert. Auch hier bildet das distale Ringelement den distalen Verankerungspunkt der Zugdrähte.

Bei der Anordnung der Zugdrähte relativ zum Arbeitskanalelement 1 können in einer weiteren Alternative am distalen Ende zwei Zugdrähte entlang des Umfangs nahe zueinander angeordnet sein (kurzer Abstand) und an der proximalen Seite am Schieber 20 jeweils um 180° Grad zueinander beabstandet sein (gleicher Abstand). Der in der Umfangsrichtung gemessene Abstand der einzelnen Zugdrähte zueinander ist also entlang des gesamten Schlauchelements 2 verändert. Wie im ersten Ausführungsbeispiel wird bei einer Zugbewegung des Schiebers 20 in die proximale Richtung der Abwinkelungsabschnitt 11 an der lateralen Seite, an der die beiden Zugdrähte distal verankert sind, nach außen abgewinkelt.

Alternativ können auch drei oder vier oder mehr Zugdrähte im erfindungsgemäßen Arbeitskanalelement 1 vorgesehen sein. Sollten drei oder mehr Zugdrähte verwendet werden, können diese auch relativ nahe beieinander an einer Seite des Umfangs am distalen Ende des Arbeitskanalelements 1 verankert sein.

Um die Steuergenauigkeit zu verbessern, können alternativ die drei oder mehr Zugdrähte so am Umfang am distalen Ende des Arbeitskanalelements 1 verankert sein, dass zwei (von dreien), drei (von vieren) etc. der Zugdrähte relativ nahe beieinander an einer Seite des Umfangs sind, und ein Zugdraht an der gegenüberliegenden Umfangsseite verankert ist. In dieser Alternative ist der Schieber 20 drehbar um das Stabelement 14 (um seine eigene Achse) vorgesehen. Durch Ziehen und Drehen des Schiebers 20 relativ zum Stabelement 14 können die jeweiligen Zugdrähte individuell unterschiedlich gespannt werden. Beispielsweise können für ein veranschaulichendes spezifisches Beispiel dieser Alternative die Zugdrähte bei 12 Uhr, 1 Uhr, 6 Uhr und 11 Uhr auf dem Umfang am distalen Ende des Arbeitskanalelements 1 verankert sein. Die Feststelleinrichtung 30 ist dann nicht als Bolzen ausgebildet, der durch den Schlitz 14A tritt, sondern z.B. als am Umfang des Stabelements 14 wirkender am Schieber 20 sitzender Klemmmechanismus.

Im Übrigen ist die Steuereinheit 20 nicht auf den Schieber oder den Hebel beschränkt. Als Steuereinheit 20 kann z.B. ein Handrad angewendet werden. Ein beliebiger Mechanismus, der Zugdrähte einzeln oder als Gruppe spannen kann, ist anwendbar.

Die Steuereinheit 20 muss auch nicht aus lediglich einem Mechanismus bestehen. Es können auch zwei oder mehr Mechanismen angewendet werden, die jeweils einem oder einer Gruppe an Zugdrähten zugewiesen sind. Dadurch kann die Steuergenauigkeit noch weiter verbessert werden. Beispielsweise kann der Schieber 20 des ersten Ausführungsbeispiels in axialer Richtung im Achsenbereich in zwei Schieberelemente getrennt sein, die je einen Seitenflügel 20C ausbilden und im Achsenbereich einander überlappen. Anstelle des Rohrabschnittes 20A hat jeder Seitenflügel 20C einen Teilrohrabschnitt 20A, von denen einer den anderen übergreift. In beiden Seitenflügeln 20C ist mindestens ein Zugdraht verankert, und die Seitenflügel 20C können relativ zueinander entlang des Stabelements 14 verschoben werden. Anstelle des Lochs 20B ist ein entsprechender sich axial erstreckender Schlitz in beiden Seitenflügeln 20C am Teilrohrabschnitt 20A vorgesehen. In dieser Alternative sind die Zugdrähte separat steuerbar.

In der vorliegenden Erfindung ist ein Zugdraht zum Steuern des Abwinkelns des distalen Endes des Arbeitskanalelements 1 beschrieben. Mit dem Begriff "Zugdraht" ist nicht nur ein Draht im allgemeinen Sinne gemeint. Als "Zugdraht" kann ein Zugseil oder eine Bowdenzugkonstruktion angewendet werden. Ferner unterliegt das Material des "Zugdrahtes" keinen Einschränkungen. Der "Zugdraht" muß lediglich in der Lage sein, eine Kraft eines beliebigen Steuermechanismus auf das distale Endes des Arbeitskanalelements 1 zu übertragen, um die Richtung und/oder den Winkel und/oder das Ausmaß des Abwinkelns des distalen Endes des Arbeitskanalelements 1 zu steuern.

Ein nicht erfindungsgemässes Arbeitskanalelement kann auch ohne Abwinkelungsabschnitt 11 ausgebildet sein. In dieser Alternative wird auch keine Steuereinheit zum Steuern des Abwinkelns benötigt. Auch ein solches Arbeitskanalelement bietet den Vorteil, dass der eigentliche Arbeitskanal des Endoskops, in welchen das Arbeitskanalelement eingeführt wird, bei der Anwendung nicht kontaminiert wird. Eine Verhinderung von Kreuzkontaminationen ist auch in dieser Alternative möglich, da für jeden Patienten ein eigener Arbeitskanal verwendet wird.

### Bezugszeichenliste

- 1: Arbeitskanalelement
- 2: Schlauch
- 11: Abwinkelungsabschnitt
- 12: Hauptschlauch
- 13: Übergangselement
- 14: Stabelement
- 14A: Schlitz
- 15: Nase
- 16: Tellerelement
- 17: Instrumentenzugang
- 20: Steuereinheit, Schieber
- 20': Steuereinheit, Hebel
- 20A: Rohrabschnitt
- 20B: Loch
- 20C: Seitenflügel
- 20D: Öffnung
- 30: Feststelleinrichtung, Drehbolzen
- 50: Montageeinrichtung
- 51: Luer-lock-Gegenstück
- 52: Schraubkappe
- 60: Gehäuseteil
- 61: Gehäusestück
- 62: Verschlusselement
- 100: Endoskop
- 101: Arbeitskanal des Endoskops
- 102: Ultraschallsensor des Endoskops
- 103: Steuereinheit des Endoskops, Stellknopf
- 104: Anschluss für Arbeitskanalelement
- 105: Griffabschnitt
- 106: Kabel
- 107: Endoskop-Abwinklungsabschnitt
- 108: Endoskopkopf
- A: Bedienteil
- B: Einführabschnitt

## Patentansprüche

1. Arbeitskanalelement (1) für ein Endoskop,
wobei das Arbeitskanalelement (1) eine Rohrform mit einem Innenumfang und einem Außenumfang aufweist, durch die ein Instrument schiebbar ist,
wobei das Arbeitskanalelement (1) daran angepasst ist, mit seiner distalen Seite voran in einen Arbeitskanal (101) eines Endoskops (100) vorübergehend eingeführt zu werden, und
wobei das Arbeitskanalelement (1) ein flexibles Schlauchelement (2) aufweist;
das Arbeitskanalelement (1) ein Übergangselement (13) an einer proximalen Seite des flexiblen Schlauchelements (2) hat, wobei das proximale Ende des flexiblen Schlauchelements (2) und das Übergangselement (13) auf gleicher Achse angeordnet sind; und
das Übergangselement (13) eine Außenwand aufweist, die lateral von einem Instrumentenzugang (17) durchdrungen ist, wobei durch den Instrumentenzugang (17) Instrumente in einen Innenkanal des Arbeitskanalelementes (1) geschoben werden können,
das Arbeitskanalelement (1) an der distalen Seite einen Abwinkelungsabschnitt (11) aufweist,
das Arbeitskanalelement (1) an der proximalen Seite eine Steuereinheit (20) zum Steuern des Abwinkelns des Abwinkelungsabschnittes (11) aufweist,
die Steuereinheit (20) so aufgebaut ist, dass sie zumindest einen im Arbeitskanalelement (1) zwischen Innenumfang und Außenumfang geführten am Abwinkelungsabschnitt verankerten Steuerdraht spannt, um das Abwinkeln des Abwinkelungsabschnittes (11) zu steuern,
die Steuereinheit (20) als Schieber aufgebaut ist, in dem das proximale Ende des Steuerdrahtes verankert ist, **dadurch gekennzeichnet, dass**
das Arbeitskanalelement (1) an der proximalen Seite eine Feststelleinrichtung (30) zum Fixieren einer abgewinkelten Stellung des distalen Abwinkelungsabschnittes aufweist, wobei die Feststelleinrichtung (30) am Schieber (20) sitzt.

2. Arbeitskanalelement (1) gemäß Anspruch 1, wobei
die Steuereinheit eine Vielzahl an separaten Steuerelementen aufweist, wobei jedem separaten Steuerelement mindestens einer der Steuerdrähte zugeordnet ist.

3. Arbeitskanalelement (1) gemäß einem der Ansprüche 1 oder 2, wobei
das Arbeitskanalelement (1) an der proximalen Seite eine Montageeinrichtung (50) aufweist, durch die das Arbeitskanalelement (1) an einem Endoskop montiert werden kann.

4. Endoskop mit einem Arbeitskanalelement (1) gemäß einem der Ansprüche 1 bis 3.

5. Verfahren zur Anwendung eines Arbeitskanalelements (1) gemäß Anspruch 1 in einem Endoskop,
wobei im Verfahren ein separates Arbeitskanalelement (1) mit seiner distalen Seite voran in einen Arbeitskanal (101) eines Endoskops (100) vorübergehend eingeführt wird, bevor das mit dem Arbeitskanalelement (1) bestückte Endoskop (100) in den Patienten zu dem erwünschten Anwendungsort eingeführt werden kann.

## Claims

1. A working channel element (1) for an endoscope,
wherein the working channel element (1) has a tubular shape which has an inner circumference and an outer circumference and through which an instrument can be pushed,
wherein the working channel element (1) is adapted to be temporarily inserted into a working channel (101) of an endoscope (100) with its distal side ahead, and
wherein the working channel element (1) comprises a flexible tube element (2);
the working channel element (1) has a transition element (13) at a proximal side of the flexible tube element (2), wherein the proximal end of the flexible tube element (2) und the transition element (13) are arranged on the same axis; and
the transition element (13) comprises an outer wall that is penetrated laterally by an instrument access (17), wherein instruments can be pushed through the instrument access (17) into an inner channel of the working channel element (1),
the working channel element (1) has a bending portion (11) on the distal side,
the working channel element (1) has, on the proximal side, a control unit (20) for controlling the bending of the bending portion (11),
the control unit (20) is configured such that it tensions at least one control wire guided in the working channel element (1) between the inner circumference and the outer circumference and anchored at the bending portion, so as to control the bending of the bending portion (11),
the control unit (20) is configured as a slider in which the proximal end of the control wire is anchored,
**characterized in that**
the working channel element (1) comprises, on the proximal side, a locking means (30) for fixing a bent position of the distal bending portion, wherein the locking means (30) is located on the slider (20).

2. The working channel element (1) according to one of claim 1, wherein
the control unit comprises a plurality of separate control elements, wherein at least one of the control wires is assigned to each separate control element.

3. The working channel element (1) according to one of claims 1 or 2, wherein
the working channel element (1) comprises, on the proximal side, a mounting means (50) by means of which the working channel element (1) can be mounted to an endoscope.

4. An endoscope comprising a working channel element (1) according to one of claims 1 to 3.

5. A method for using a working channel element (1) according to claim 1 in an endoscope,
wherein in the method, a separate working channel element (1) is, with its distal side ahead, temporarily inserted into a working channel (101) of an endoscope (100), before the endoscope (100) equipped with the working channel (101) can be inserted into the patient to the desired application site.

## Revendications

1. Élément (1) à canal de travail pour un endoscope,
l'élément (1) à canal de travail ayant une forme tubulaire avec une circonférence intérieure et une circonférence extérieure, à travers laquelle un instrument est apte à être poussé,
l'élément (1) à canal de travail étant adapté pour être inséré temporairement, avec son côté distal en premier, dans un canal de travail (101) d'un endoscope (100), et
l'élément (1) à canal de travail comprenant un élément de tuyau flexible (2) ;
l'élément (1) à canal de travail ayant un élément de transition (13) sur un côté proximal de l'élément de tuyau flexible (2), l'extrémité proximale de l'élément de tuyau flexible (2) et l'élément de transition (13) étant agencés sur le même axe ; et
l'élément de transition (13) présente une paroi extérieure qui est traversée latéralement par un accès (17) pour instruments, des instruments étant aptes à être poussés dans un canal intérieur de l'élément (1) à canal de travail à travers l'accès (17) pour instruments,
l'élément (1) à canal de travail présente une portion d'angulation (11) sur le côté distal,
l'élément (1) à canal de travail présente, sur le côté proximal, une unité de commande (20) pour commander l'angulation de la portion d'angulation (11),
l'unité de commande (20) est conçue de telle sorte qu'elle tend au moins un fil de commande guidé dans l'élément (1) à canal de travail entre la périphérie intérieure et la périphérie extérieure et ancré sur la portion d'angulation, afin de commander l'angulation de la portion d'angulation (11),
l'unité de commande (20) est conçue sous la forme d'un coulisseau dans lequel est ancrée l'extrémité proximale du fil de commande, **caractérisé en ce que**
l'élément (1) à canal de travail présente sur le côté proximal un dispositif de blocage (30) pour fixer une position angulée de la portion d'angulation distale, le dispositif de blocage (30) étant placé sur le coulisseau (20).

2. Élément (1) à canal de travail selon la revendication 1, dans lequel
l'unité de commande comprend une pluralité d'éléments de commande séparés, au moins un des fils de commande étant associé à chaque élément de commande séparé.

3. Élément (1) à canal de travail selon l'une des revendications 1 ou 2, dans lequel
l'élément (1) à canal de travail présente sur le côté proximal un dispositif de montage (50) par lequel l'élément (1) à canal de travail est apte à être monté sur un endoscope.

4. Endoscope comprenant un élément (1) à canal de travail selon l'une des revendications 1 à 3.

5. Procédé d'utilisation d'un élément (1) à canal de travail selon la revendication 1 dans un endoscope,
dans lequel, dans le procédé, un élément (1) à canal de travail séparé est introduit temporairement, avec son côté distal en premier, dans un canal de travail (101) d'un endoscope (100), avant que l'endoscope (100) équipé de l'élément (1) à canal de travail ne soit introduit dans le patient jusqu'au site d'application souhaité.
